(19) Europäisches Patentamt
European Patent Office
Office européen des brevets

(11) Publication number: **0 035 284**
**B1**

(12) **EUROPEAN PATENT SPECIFICATION**

(45) Date of publication of patent specification: **13.07.83**

(51) Int. Cl.³: **C 07 C 121/76,** C 07 D 295/14

(21) Application number: **81102641.8**

(22) Date of filing: **17.08.79**

(60) Publication number of the earlier application in accordance with Art. 76 EPC: **0016277**

(54) **Novel 2-benzoyl-3-substituted-2-alkenonitriles and process for their preparation.**

(30) Priority: **03.11.78 US 957595**
**13.12.78 US 968899**

(43) Date of publication of application:
**09.09.81 Bulletin 81/36**

(45) Publication of the grant of the patent:
**13.07.83 Bulletin 83/28**

(84) Designated Contracting States:
**BE CH DE FR GB IT NL SE**

(56) References cited:
**DE - A - 2 330 913**
**FR - A - 1 535 809**
**FR - A - 2 023 474**

**JOURNAL OF MEDICINAL CHEMISTRY, vol. 22, no. 11, November 1979 DAVID N. RIDGE et al.: "Potential Antiarthritic Agents. 2. Benzoylacetonitriles and beta-Aminocinnamonitriles", pages 1385—1389.**

(73) Proprietor: **AMERICAN CYANAMID COMPANY**
**Berdan Avenue**
**Wayne New Jersey 06904 (US)**

(72) Inventor: **Hanifin, John William, Jr.**
**2 Sunset Terrace**
**Suffern New York (US)**
Inventor: **Ridge, David Nelson**
**Old Mountain Road**
**Upper Grandview New York (US)**

(74) Representative: **Allam, Peter Clerk et al,**
**LLOYD WISE, TREGEAR & CO. Norman House**
**105-109 Strand**
**London WC2R 0AE (GB)**

(56) References cited:
**SYNTHESIS 1973, no. 1 January, pages 1—70, "International Journal of Methods in Synthetic Organic Chemistry" Georg Thieme Verlag, Stuttgart.**

**EP 0 035 284 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Novel 2-benzoyl-3-substituted-2-alkenonitriles and process for their preparation

This invention relates to novel substituted 2-benzoyl-3-substituted-2-alkenonitriles, which are useful as intermediates for the preparation of certain substituted *cis*-2-benzoyl-3-hydroxy-2-alkenonitriles which possess antiinflammatory activity, as described in our European Patent Application 0016277A (Serial No. 79301687.4) from which the present application is divided.

According to the invention there is provided substituted 2-benzoyl-3-substituted-2-alkenonitriles of the formula:

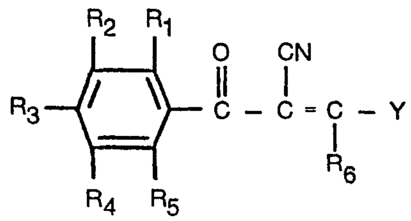

I

wherein Y represents the group OR or the group

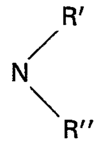

wherein $R_1$, $R_2$, $R_3$, $R_4$ and $R_5$ are each individually selected from hydrogen, halogen, alkyl having from 1 to 4 carbon atoms, alkoxy having from 1 to 4 carbon atoms, trifluoromethyl and trichloromethyl, with the proviso that at least two of $R_1$, $R_2$, $R_3$, $R_4$ and $R_5$ must be hydrogen; $R_6$ is alkyl having from 1 to 4 carbon atoms; R is alkyl having from 1 to 4 carbon atoms; and R′ and R″ are each hydrogen or alkyl having from 1 to 4 carbon atoms or R′ and R″ taken together with the associated nitrogen is pyrrolidino, piperidino, morpholino, thiomorpholino or N-methyl piperazino. Halogen is exemplified by fluoro, chloro and bromo.

In an embodiment of the invention Y is OR, all of $R_1$, $R_2$, $R_3$, $R_4$ and $R_5$ are hydrogen and R and $R_6$ are as defined above. In another embodiment of the invention, in the compounds of Formula I, $R_6$ is methyl, Y is OR and R, $R_1$, $R_2$, $R_3$, $R_4$ and $R_5$ are as defined above but at least one of $R_1$, $R_2$, $R_3$, $R_4$ and $R_5$ is other than hydrogen. In a further embodiment Y is —NR′R″, all of $R_1$, $R_2$, $R_3$, $R_4$ and $R_5$ are hydrogen, and $R_6$, R′ and R″ are as defined above. In a still further embodiment, Y is OR wherein R is methyl or ethyl; three of the radicals $R_1$, $R_2$, $R_3$, $R_4$ and $R_5$ are methoxy and the remaining of said radicals are hydrogen; or one of said $R_1$—$R_5$ radicals is fluoro, bromo, trifluoromethyl or chloro, the remaining being hydrogen; and $R_6$ is methyl or ethyl. In a yet further embodiment Y is

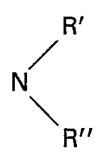

wherein R′ and R″ are each methyl, ethyl, n-butyl or hydrogen, or taken together are piperidyl; three of the radicals $R_1$, $R_2$, $R_3$, $R_4$ and $R_5$ are methoxy and the remaining of said radicals are hydrogen; or one of said radicals $R_1$—$R_5$ is methyl, another is chloro and the remaining being hydrogen; or two of said radicals $R_1$—$R_5$ are fluoro, the remaining being hydrogen; or one of said radicals $R_1$—$R_5$ is fluoro or chloro, the remaining being hydrogen; and $R_6$ is methyl or ethyl.

The compounds of Formula I are new organic compounds and, more particularly, novel substituted 2-benzoyl-3-alkoxy-2-alkenonitriles (i) and novel substituted 2-benzoyl-3-amino-2-alkenonitriles (ii) which may be represented by the following structural formulae:

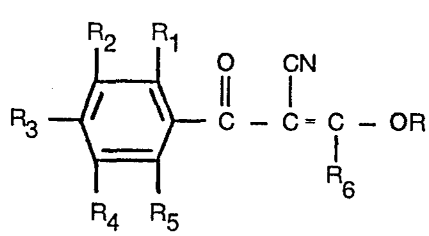

(i)

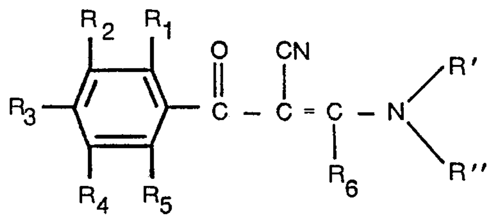

(ii)

wherein the groups are as defined above for Formula I.

The novel compounds of Formula I are obtainable as crystalline materials having characteristic melting points and absorption spectra. They are appreciably soluble in many organic solvents such as lower alkanols, acetone, ethyl acetate, and the like but are generally insoluble in water.

The preparation of the novel compounds of the present invention and their conversion to useful final products is set forth in the following reaction scheme wherein $R_1$, $R_2$, $R_3$, $R_4$, $R_5$, $R_6$, R, R′ and R″ are as hereinbefore defined for Formula I.

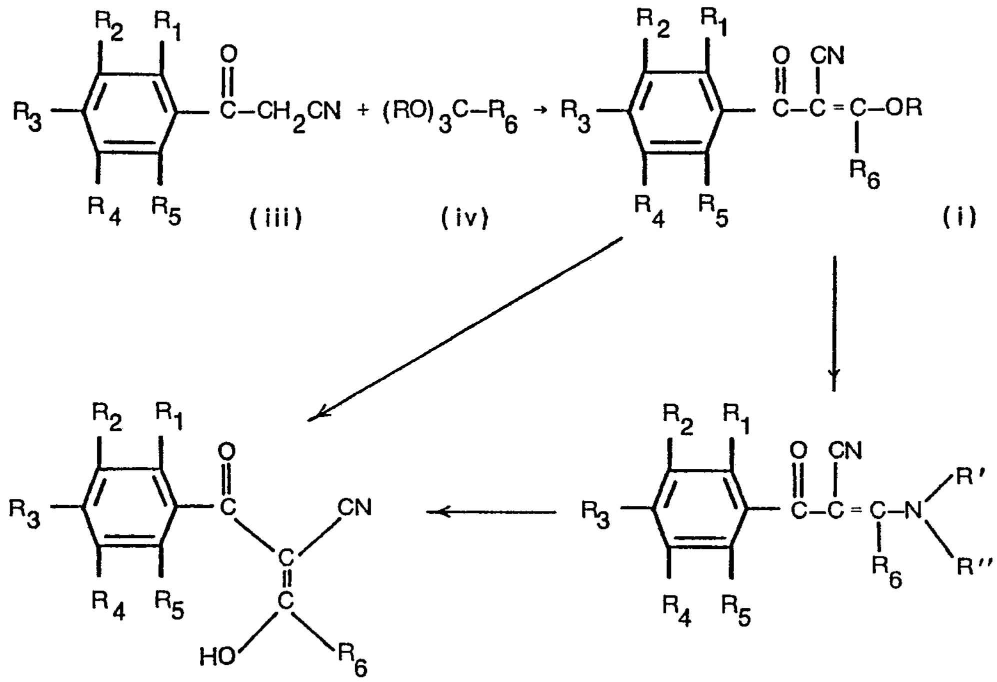

(iii) (iv) (i) (v) (ii)

In accordance with this reaction scheme, an appropriately substituted benzoylacetonitrile (iii) is condensed with a trialkyl orthoester (iv) in refluxing acetic anhydride. Evaporation of by-products and excess acetic anhydride *in vacuo* and purification of the residue under anhydrous conditions provides the corresponding substituted 2-benzoyl-3-alkoxy-2-alkenonitrile (i) of this invention. Treatment of (i) with ammonia or a primary or secondary amine of the formula:

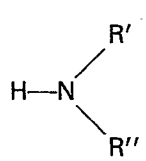

at steam bath temperature under pressure in a sealed vessel for 8—12 hours then provides the substituted 2-benzoyl-3-amino-2-alkenonitriles (ii) of this invention. Both of these intermediates (i) and (ii) may be hydrolyzed under acidic conditions to provide the anti-inflammatory products (v) within the scope of the parent application. The compounds (ii) wherein R′ and R″ are both methyl may also be prepared by treatment of an appropriately substituted benzoylacetonitrile (iii) with N,N-dimethylacetamide dimethylacetal at low temperature in chloroform, methylene chloride or even as a neat mixture of reagents.

The invention is illustrated by the Examples which follow:

Example 1

3-Dimethylamino-2-(p-fluorobenzoyl)crotononitrile

To a solution of 1.6 g of p-fluorobenzoylacetonitrile[Pihl *et al.,* Reakts. Sposobnost Org. Soedin. Tartu. Gos. Univ., *5* (1), 27, (1968)] in 30 ml. of chloroform, cooled to −10°C is added 1.4 g of N,N-dimethylacetamide dimethylacetal. The reaction mixture is stirred at −10°C for 2 hours and then evaporated *in vacuo* to an oil. This oil is dissolved in 150 ml of benzene and filtered through Magnesol®. The filtrate is evaporated to 50 ml and petroleum ether is added to effect crystallization. The product is collected by filtration and then recrystallized from benzene-petroleum ether with charcoal treatment giving the desired product.

Example 2

2-(p-Fluorobenzoyl)-3-methoxycrotononitrile

A solution containing 55.8 g (0.34 mole) of *p*-fluorobenzoylacetonitrile, 41 g (0.34 mole) of trimethyl orthoacetate, and 90 g (0.88 mole) of acetic anhydride is heated to reflux for 5 hours. The excess solvent and volatile by-products are then removed by vacuum distillation and the residue is recrystallized from diethyl ether. Alternatively, the distillation residue may be distilled over on a Kugelrohr apparatus at 160°C/0.25 mm to provide a viscous oil which may be crystallized from diethyl ether to provide the pure title compound.

Example 3

3-Amino-2-(p-fluorobenzoyl)crotononitrile

Approximately 50 ml of liquid ammonia is condensed in a three-neck flask cooled to −78° in a dry ice-acetone bath. To this is added 200 mg of sodium metal, followed by 100 mg of ferric chloride hexahydrate. When the blue color had vanished, 6.2 g of 2-(*p*-fluorobenzoyl)-3-methoxycrotononitrile is added. After stirring for one hour, the reaction is allowed to warm and the solvent evaporate. The residue is dissolved in hot ethyl acetate and filtered through Celite®. The filtrate is concentrated on the steam bath and diluted with hexane. Upon cooling, the crystalline product precipitates, is filtered and dried.

Example 4

2-(p-Fluorobenzoyl)-3-methylaminocrotononitrile

In the glass liner of a stainless steel bomb cooled to −78° is condensed 10 ml of methylamine. To this is added 0.5 ml of 2M *n*-butyllithium (in hexane), followed by 5.0 g of 2-(*p*-fluorobenzoyl)-3-methoxycrotononitrile. The bomb is sealed and heated on a steam bath overnight. The bomb is then cooled, opened and the contents evaporated. The residue is dissolved in chloroform, passed through a Magnesol® pad and the filtrate is evaporated. The residue is recrystallized from chloroformhexane yielding the title compound.

Example 5

2-(p-Fluorobenzoyl)-3-butylaminocrotononitrile

A sample of 2.5 g of 2-(*p*-fluorobenzoyl)-3-methoxy crotononitrile is dissolved in 10 ml of *n*-butylamine. The solution is sealed in a stainless steel bomb and heated on a steam bath overnight. The bomb is then opened and the solvent evaporated under reduced pressure. A greenish oil is obtained which is distilled on a Kugelrohr apparatus at 200°C/0.1 mm to obtain the yellow oily product.

Example 6

2-(p-Fluorobenzoyl)-3-dimethylaminocrotononitrile

To a solution of 14.5 g (0.10 m) of (*p*-fluorobenzoyl)acetonitrile in 100 ml of chloroform, cooled in an ice-salt bath, was added 13.3 g (0.1 m) of N,N-dimethylacetamide dimethylacetal in 20 ml chloroform. The reaction mixture was stirred in the ice-salt bath for 2 hours, then evaporated *in vacuo* to an orange oil. The oil was dissolved in 150 ml benzene and passed through Magnesol®. The filtrate was evaporated and the oil thus obtained was dissolved in 50 ml benzene to which petroleum ether was added until the solution became cloudy. The precipitate which formed upon cooling was collected. The solid was recrystallized twice from benzene-petroleum ether, with charcoal treatment to yield 5.8 g (27%) of light yellow crystals, m.p. 90°—92°C.

Example 7

2-(p-Fluorobenzoyl)-3-methoxy-2-pentenonitrile

A solution containing 30 g of (*p*-fluorobenzoyl)acetonitrile, 36.4 g of trimethylorthopropionate and 57 g of acetic anhydride is heated in an oil bath at 130° for 3 hours. The ethanol and excess acetic anhydride is distilled off and the residue is diluted with chloroform. The chloroform solution is passed quickly through Magnesol® and concentrated, then cooled to produce the title product.

Example 8

2-(p-Fluorobenzoyl)-3-N-piperidylcrotononitrile

To a solution of 5.0 g of 2-(*p*-fluorobenzoyl)-3-methoxycrotononitrile in 50 ml of dry tetrahydrofuran is added 5 ml of piperidine. The reaction mixture is heated to reflux for 2 hours, cooled and evaporated *in vacuo*. The residue is recrystallized from chloroform/hexane to provide the crystalline title compound.

Example 9

2-(p-Fluorobenzoyl)-3-diethylaminocrotononitrile

A volume of 10 ml of diethylamine is condensed in a glass tube at —78°C. A sample of 5 g of 2-(*p*-fluorobenzoyl)-5-methoxycrotononitrile is added, the tube is sealed in stainless steel bomb and heated on a steam bath overnight. The bomb is then cooled, opened, and the contents evaporated. The residue was recrystallized from chloroform to provide the title compound.

Example 10

2-(p-Fluorobenzoyl)-3-ethoxycrotononitrile

A solution of 16.3 g of *p*-fluorobenzoylacetonitrile and 18.3 ml of triethyl orthoacetate in 35 ml of acetic anhydride is heated on a steam bath for 2.5 hours. The solvents are removed *in vacuo* and the residue is distilled at 165°—167°C/300 $\mu$. Upon cooling, this distillate solidifies and is recrystallized from methylene chloride/hexane to give the desired 2-(*p*-fluorobenzoyl)-3-ethoxycrotononitrile, m.p. 77°/89°C.

Example 11

2-Benzoyl-3-methylaminocrotononitrile

In the glass liner of a stainless steel bomb cooled to —78°C is condensed 10 ml of methylamine. To this is added 0.5 ml of 2M *n*-butyllithium (in hexane), followed by 5.0 g of 2-benzoyl-3-methoxycrotononitrile. The bomb is sealed and heated on a steam bath overnight. The bomb is then cooled, opened and the contents evaporated. The residue is dissolved in chloroform, passed through a Magnesol® pad and the filtrate is evaporated. The residue is recrystallized from chloroform-hexane yielding the title compound.

Example 12

2-Benzoyl-3-butylaminocrotononitrile

A sample of 2.5 g of 2-benzoyl-3-methoxycrotononitrile is dissolved in 10 ml of *n*-butylamine. The solution is sealed in a stainless steel bomb and heated on a steam bath overnight. The bomb is then opened and the solvent evaporated under reduced pressure. A greenish oil is obtained which is distilled on a Kugelrohr apparatus at 200°C/0.1 mm to obtain the yellow oily product.

Example 13

2-Benzoyl-3-dimethylaminocrotononitrile

To a solution of 14.5 g (0.10 m) of benzoylacetonitrile in 100 ml of chloroform, cooled in an ice-salt bath, was added 13.3 g (0.1 m) of N,N-dimethylacetamide dimethylacetal in 20 ml chloroform. The reaction mixture was stirred in the ice-salt bath for 2 hours, then evaporated *in vacuo* to an orange oil. The oil was dissolved in 150 ml benzene and passed through Magnesol®. The filtrate was evaporated and the oil thus obtained was dissolved in 50 ml benzene to which petroleum ether was added until the solution became cloudy. The precipitate which formed upon cooling was collected. The solid was recrystallized twice from benzene petroleum ether, with charcoal treatment to yield 5.8 g (27%) of light yellow crystals, m.p. 105°—106°C.

Example 14

2-Benzoyl-3-methoxy-2-pentenonitrile

A solution containing 30 g of benxoylacetonitrile, 36.4 g of triethylorthopropionate and 57 g of acetic anhydride is heated in an oil bath at 130° for 3 hours. The ethanol and excess acetic anhydride is distilled off and the residue is diluted with chloroform. The chloroform solution is passed quickly through Magnesol® and concentrated, then cooled to produce the title product. Similarly prepared is 2-benzoyl-3-methoxy-2-hexenonitrile and 2-benzoyl-3-methoxy-2-heptenonitrile.

Example 15

2-Benzoyl-3-N-piperidylcrotononitrile

To a solution of 5.0 g of 2-benzoyl-3-methoxycrotononitrile in 50 ml of dry tetrahydrofuran is added 5 ml of piperidine. The reaction is heated to reflux for 2 hours, cooled and evaporated *in vacuo*. The residue is re-crystallized from chloroform/hexane to provide the crystalline title compound.

Example 16

2-Benzoyl-3-dimethylaminocrotononitrile

A volume of 10 ml of diethylamine is condensed in a glass tube at —78°C. A sample of 5 g of 2-benzoyl-5-methoxycrotononitrile is added, the tube is sealed in a stainless steel bomb and heated on a steam bath overnight. The bomb is then cooled, opened, and the contents evaporated. The residue was recrystallized from chloroform to provide the title compound.

Similarly prepared were 2-benzoyl-3-diethylaminocrotononitrile and 2-benzoyl-3-diisopropylaminocrotononitrile.

Example 17

2-(p-Fluorobenzoyl)-3-dimethylaminocrotononitrile

Dimethylamine is bubbled into a solution of 2-(*p*-fluorobenzoyl)-3-ethoxycrotononitrile in 50 ml of diethyl ether for 15 minutes. After stirring at room temperature for one hour, the reaction mixture is allowed to evaporate to dryness and the resulting solid is recrystallized from methylene chloride/hexane to give 2-(*p*-fluorobenzoyl)-3-dimethylaminocrotononitrile.

**Claims**

1. Substituted 2-benzoyl-3-substituted-2-alkenonitriles of the formula:

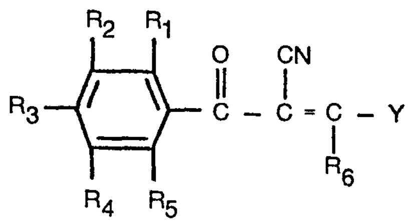

I

wherein Y represents the group OR or the group

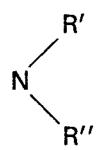

wherein $R_1$, $R_2$, $R_3$, $R_4$ and $R_5$ are each individually selected from hydrogen, halogen, alkyl having from 1 to 4 carbon atoms, alkoxy having from 1 to 4 carbon atoms, trifluoromethyl or trichloromethyl, with the proviso that at least two of $R_1$, $R_2$, $R_3$, $R_4$ and $R_5$ must be hydrogen; $R_6$ is alkyl having from 1 to 4 carbon atoms; R is alkyl having from 1 to 4 carbon atoms; and R′ and R″ are each hydrogen or alkyl having from 1 to 4 carbon atoms or R′ and R″ taken together with the associated nitrogen is pyrrolidino, piperidino, morpholino, thiomorpholino or N-methylpiperazino.

2. A compound according to Claim 1, wherein Y is

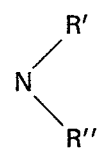

and all of $R_1$, $R_2$, $R_3$, $R_4$ and $R_5$ are hydrogen.

3. A compound according to Claim 1, wherein Y is OR and all of $R_1$, $R_2$, $R_3$, $R_4$ and $R_5$ are hydrogen.

4. A compound according to Claim 1, wherein $R_6$ is methyl, Y is OR and at least one of $R_1$, $R_2$, $R_3$, $R_4$ and $R_5$ is other than hydrogen.

5. A compound according to Claim 1, wherein Y, is OR wherein R is methyl or ethyl, and three of the radicals $R_1$, $R_2$, $R_3$, $R_4$ and $R_5$ are methoxy and the remaining of said radicals are hydrogen; or one of said $R_1$—$R_5$ radicals is fluoro, bromo, trifluoromethyl or chloro, the remaining being hydrogen; and $R_6$ is methyl or ethyl.

6. A compound according to Claim 1, wherein Y is

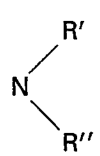

wherein R′ and R″ are each methyl, ethyl, n-butyl or hydrogen, or taken together are piperidyl, and three of the radicals $R_1$, $R_2$, $R_3$, $R_4$ and $R_5$ are methoxy and the remaining of said radicals are hydrogen; or one of said radicals $R_1$—$R_5$ is methyl, another is chloro and the remaining being hydrogen; or two of said radicals $R_1$—$R_5$ are fluoro, the remaining being hydrogen; or one of said radicals $R_1$—$R_5$ is fluoro or chloro, the remaining being hydrogen; and $R_6$ is methyl or ethyl.

7. A method of producing compounds of Formula I as claimed in Claim 1, which comprises reacting in refluxing acetic anhydride an acetonitrile of the formula:

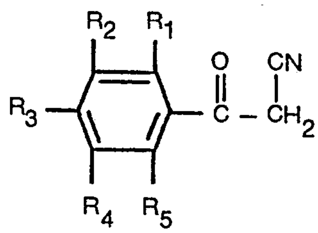

with a compound of the formula:

$$R_6—C(OR)_3$$

wherein R, $R_1$, $R_2$, $R_3$, $R_4$, $R_5$ and $R_6$ are as defined for Formula I, to produce a compound of Formula I in which Y is OR; and where desired, treating the compound where Y is OR with ammonia or a primary or secondary amine, thereby producing a compound of Formula I in which Y is the group

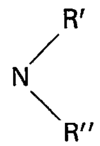

wherein R′ and R″ are as defined for Formula I.

**Revendications**

1. Benzoyl-2 alcène-2 onitriles substitués en position 3 de formule

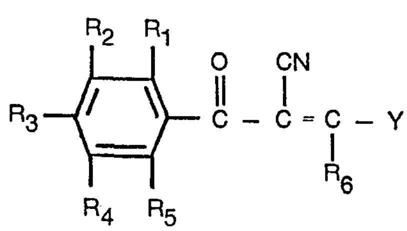

I

où Y représente le groupe OR ou le groupe

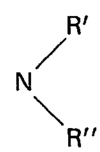

où $R_1$, $R_2$, $R_3$, $R_4$ et $R_5$ sont chacun individuellement choisis parmi un hydrogène, un halogène, un alkyle ayant 1 à 4 atomes de carbone, un alcoxy ayant 1 à 4 atomes de carbone, un trifluorométhyle ou un trichlorométhyle, sous réserve qu au moins deux de $R_1$, $R_2$, $R_3$, $R_4$ et $R_5$ soient un hydrogène; $R_6$ est un alkyle ayant 1 à 4 atomes de carbone; R est un alkyle ayant 1 à 4 atomes de carbone; et R′ et R″ sont chacun un hydrogène ou un alkyle ayant 1 à 4 atomes de carbone ou R′ et R″ pris ensemble avec l'azote associé sont un pyrrolidino, un pipéridino, un morpholino, un thiomorpholino ou un N-méthylpipérazino.

2. Un composé selon la revendication 1 où Y est

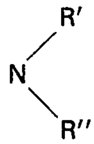

et $R_1$, $R_2$, $R_3$, $R_4$ et $R_5$ sont tous un hydrogène.

3. Un composé selon la revendication 1, où Y est OR et $R_1$, $R_2$, $R_3$, $R_4$ et $R_5$ sont tous un hydrogène.

4. Un composé selon la revendication 1 où $R_6$ est un méthyle, Y est OR et au moins un de $R_1$, $R_2$, $R_3$, $R_4$ et $R_5$ est autre qu'un hydrogène.

5. Un composé selon la revendication 1 où Y est OR où R est un méthyle ou un éthyle et trois des radicaux $R_1$, $R_2$, $R_3$, $R_4$ et $R_5$ sont un méthoxy et ceux qui restent desdits radicaux sont un hydrogène; ou un desdits radicaux est un fluoro, un bromo, un trifluorométhyl ou un chloro, ceux qui restent étant un hydrogène et $R_6$ est un méthyle ou un éthyle.

6. Un composé selon la revendication 1 où Y est

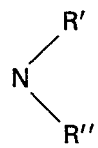

où R' et R'' sont chacun un méthyle, un éthyle, un n-butyle ou un hydrogène ou, pris ensemble, sont un pipéridyle, et trois des radicaux $R_1$, $R_2$, $R_3$, $R_4$ et $R_5$ sont un méthoxy et ceux qui restent desdits radicaux sont un hydrogène; ou un desdits radicaux $R_1$—$C_5$ est un méthyle, un autre est un chloro, et ceux qui restent sont un hydrogène; ou deux desdits radicaux $R_1$—$R_5$ sont un fluoro, ceux qui restent étant un hydrogène; ou un desdits radicaux $R_1$—$R_5$ est un fluoro ou un chloro, ceux qui restent étant un hydrogène; et $R_6$ est un méthyle ou un éthyle.

7. Un procédé pour produire des composés de formule I comme revendiqué dans la revendication 1, qui comprend la réaction dans l'anhydride acétique à reflux d'un acétonitrile de formule:

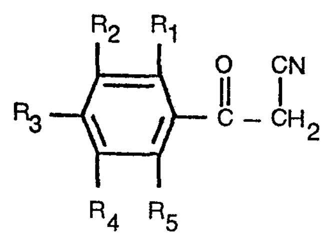

avec un composé de formule:

$$R_6—C(OR)_3$$

où R, $R_1$, $R_2$, $R_3$, $R_4$, $R_5$ et $R_6$ sont comme définis pour la formule I, pour produire un composé de formule I où Y est OR; et, lorsqu'on le desiré, le traitement du composé où Y est OR avec l'ammoniac ou une amine primaire ou secondaire, pour produire un composé de formule I où Y est le groupe

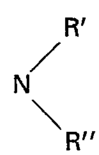

où R' et R'' sont comme définis pour la formule I.

**Patentansprüche**

1. Substituierte 2-Benzoyl-3-subst.-2-alkenonitrile der Formel

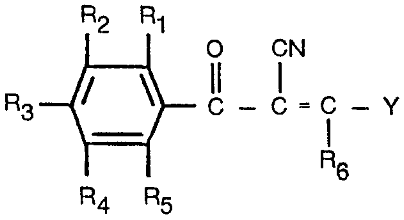

I

wobei Y für die Gruppe OR oder die Gruppe

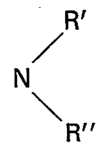

steht, $R_1$, $R_2$, $R_3$, $R_4$ und $R_5$ jeweils unabhängig voneinander ausgewählt sind unter Wasserstoff, Halogen, Alkyl mit 1 bis 4 Kohlenstoffatomen, Alkoxy mit 1 bis 4 Kohlenstoffatomen, Trifluormethyl oder Trichlormethyl mit der Maßgabe, daß wenigstens zwei der Reste $R_1$, $R_2$, $R_3$, $R_4$ und $R_5$ Wasserstoff sein müssen; $R_6$ für Alkyl mit 1 bis 4 Kohlenstoffatomen steht; R für Alkyl mit 1 bis 4 Kohlenstoffatomen steht und wobei R′ und R″ jeweils Wasserstoff oder Alkyl mit 1 bis 4 Kohlenstoffatomen bedeuten oder R′ und R″ zusammen mit dem assoziierten Stickstoff für Pyrrolidino, Piperidino, Morpholino, Thiomorpholino oder N-Methylpiperazino stehen.

2. Verbindung nach Anspruch 1, dadurch gekennzeichnet, daß Y für

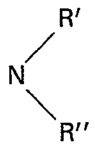

steht und alle $R_1$, $R_2$, $R_3$, $R_4$ und $R_5$ für Wasserstoff stehen.

3. Verbindung nach Anspruch 1, dadurch gekennzeichnet, daß Y für OR steht und alle Reste $R_1$, $R_2$, $R_3$, $R_4$, und $R_5$ für Wasserstoff stehen.

4. Verbindung nach Anspruch 1, dadurch gekennzeichnet, daß $R_6$ für Methyl steht, Y für OR steht und wenigstens einer der Reste $R_1$, $R_2$, $R_3$, $R_4$ und $R_5$ nicht für Wasserstoff steht.

5. Verbindung nach Anspruch 1, dadurch gekennzeichnet, daß Y für OR steht, wobei R Methyl oder Äthyl bedeutet, und drei der Reste $R_1$, $R_2$, $R_3$, $R_4$ und $R_5$ für Methoxy stehen und die übrigen Reste Wasserstoff sind; oder einer der genannten Reste für Fluor, Brom, Trifluormethyl oder Chlor steht, wobei die übrigen für Wasserstoff stehen; und $R_6$ für Methyl oder Äthyl steht.

6. Verbindung nach Anspruch 1, dadurch gekennzeichnet, daß Y für

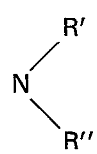

steht, wobei R′ und R″ jeweils Methyl, Äthyl, n-Butyl oder Wasserstoff bedeuten oder zusammengefaßt Piperidyl bedeuten und drei der Reste $R_1$, $R_2$, $R_3$, $R_4$ und $R_5$ für Methoxy stehen und die übrigen der Reste für Wasserstoff stehen; oder einer der Reste $R_1$ bis $R_5$ für Methyl steht, ein weiterer für Chlor steht und die übrigen für Wasserstoff stehen; oder zwei der Reste $R_1$ bis $R_5$ für Fluor stehen und die übrigen für Wasserstoff stehen; oder einer der Reste $R_1$ bis $R_5$ für Fluor oder Chlor steht und die übrigen für Wasserstoff stehen; und $R_6$ für Methyl oder Äthyl steht.

7. Verfahren zur Herstellung von Verbindungen der Formel I gemäß Anspruch 1, dadurch gekennzeichnet, daß man in refluxierendem Essigsäureanhydrid ein Acetonitril der Formel

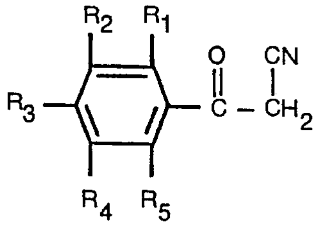

mit einer Verbindung der Formel

$R_6—C(OR)_3$

wobei R, $R_1$, $R_2$, $R_3$, $R_4$, $R_5$ und $R_6$ die für Formel I angegebenen Bedeutungen haben, zur Herstellung einer Verbindung der Formel I umsetzt, bei der Y für OR steht; und man, falls erwünscht, die Verbindung, bei der Y für OR steht, mit Ammoniak oder einem primären oder sekundären Amin behandelt, um auf diese Weise eine Verbindung der Formel I herzustellen, bei der Y für die Gruppe

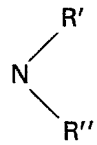

steht, wobei R' und R'' die für Formel I angegebenen Bedeutungen haben.